# EUROPEAN PATENT APPLICATION

(11) **EP 4 152 342 A1**
(43) Date of publication of application: **22.03.2023**
(21) Application number: 21197594.1
(22) Date of filing: 20.09.2021
(51) Int. Cl.: G16H 50/20

(54) **COMPUTER IMPLEMENTED METHOD AND SYSTEM FOR PROVIDING MEDICAL DECISION SUPPORT AND TRAINING METHOD**

(71) Applicant: BIOTRONIK SE & Co. KG, 12359 Berlin (DE)
(72) Inventor: DÖRR, Thomas, 12437 Berlin (DE); MÜLLER, Jens, 14195 Berlin (DE)
(74) Representative: Biotronik Corporate Services SE

(57) **Abstract**

The invention relates to a computer implemented method for providing medical decision support, comprising the steps of providing (S1) a first data set (DS1) comprising pre-acquired medical data (D) captured by at least one sensor of a medical device (10), applying (S2) a machine learning algorithm (A), in particular an artificial neural network, to the pre-acquired medical data (D), wherein the machine learning algorithm (A) comprises a first algorithm component (A1) hosted in an IT-environment of a health care provider and a second algorithm component (A2) hosted in an IT-environment of an IT-service provider, said first algorithm component (A1) and said second algorithm component (A2) being linked via a data interface (12), and outputting (S3) a second data set (DS2) representing medical decision support data (SD) by the machine learning algorithm (A). The invention further relates to a corresponding system (1) and training method.

## Description

The invention relates to a computer implemented method for providing medical decision support and a computer implemented method for providing a trained machine learning algorithm configured to provide medical decision support.

Furthermore, the invention relates to a system for providing medical decision support.

A variety of medical decision support systems exist at present, some of which employ machine learning algorithms. One of the issues of such systems is data protection of patient medical data.

IT-service providers offering data analysis solutions do not always have access to patient medical data in order to process said patient medical data in their systems in all regions of the world depending on local rules and regulations. In some regions patient data is not allowed to be processed outside of a healthcare providers IT environment such as a hospital information system.

Hence, the IT-service provider in these cases must hand over the data analysis solution to the healthcare provider in order for it to be operated at the healthcare provider's site. This however poses a danger for the IT-service provider in that algorithms could be subject to theft or reverse engineering at the healthcare provider's site or possibly be developed further without consent of the IT-service provider.

A known solution to protect machine learning algorithms from theft is to run said algorithms only in the cloud of the IT-service provider and to send to the healthcare provider only the results of an analysis performed by the machine learning algorithm.

The disadvantage of said known solution is that the execution of the machine learning algorithm in the cloud requires uploading of sensitive patient data, which in many cases is not possible for privacy reasons. This severely limits the application of the machine learning algorithms.

It is therefore an object of the present invention to provide an improved method capable of securing machine learning algorithms for providing medical decision support while at the same time meeting local rules and regulations for data protection of patient medical data.

The object is solved by a computer implemented method for providing medical decision support having the features of claim 1.

Moreover, the object is solved by a computer implemented method for providing a trained machine learning algorithm configured to provide medical decision support having the features of claim 12.

Furthermore, the object is solved by a system for providing medical decision support having the features of claim 14.

Further developments and advantageous embodiments are defined in the dependent claims.

The present invention provides a computer implemented method for providing medical decision support. The method comprises providing a first data set comprising pre-acquired medical data captured by at least one sensor of a medical device.

Furthermore, the method comprises applying a machine learning algorithm, in particular an artificial neural network, to the pre-acquired medical data, wherein the machine learning algorithm comprises a first algorithm component hosted in an IT-environment of a health care provider and a second algorithm component hosted in an IT-environment of an IT-service provider, said first algorithm component and said second algorithm component being linked via a data interface.

In addition, the method comprises outputting a second data set representing medical decision support data by the machine learning algorithm.

Moreover, the present invention provides a computer implemented method for providing a trained machine learning algorithm configured to provide medical decision support. The method comprises receiving a first training data set comprising pre-acquired medical data captured by at least one sensor of a medical device.

Furthermore, the method comprises receiving a second training data set representing medical decision support data, and training the machine learning algorithm by an optimization algorithm which calculates an extreme value of a loss function for determining medical decision support data from the pre-acquired medical data.

In addition, the present invention provides a system for providing medical decision support. The system comprises at least one sensor of a medical device providing a first data set comprising pre-acquired medical data.

Furthermore, the system comprises means for applying a machine learning algorithm, in particular an artificial neural network, to the pre-acquired medical data, wherein the machine learning algorithm comprises a first algorithm component hosted in an IT-environment of a health care provider and a second algorithm component hosted in an IT-environment of an IT-service provider, said first algorithm component and said second algorithm component being linked via a data interface, and means for outputting a second data set representing medical decision support data.

An idea of the present invention is to prevent intellectual property theft in an AI analysis that must be performed at a health care providers IT infrastructure in order to process sensitive patient data.

The machine learning algorithm is hence split up in such a way that one part of the algorithms runs at a health care providers IT infrastructure, namely the part that processes patientspecific data and pre-processes the patient data localized at the health care providers' site.

This pre-processed data is then transmitted to the second part of the AI system, which runs on a server system of the IT service provider. This provides the advantage that with part of the algorithm located at the health care providers site, misuse can no longer take place, since the other part of the algorithm is not localized there.

In addition, computing resources at the health care providers IT infrastructure are not excessively used, but only the necessary part of the IT infrastructure is engaged.

Machine learning algorithms are based on using statistical techniques to train a data processing system to perform a specific task without being explicitly programmed to do so. The goal of machine learning is to construct algorithms that can learn from data and make predictions. These algorithms create mathematical models that can be used, for example, to classify data or to solve regression type problems.

According to an aspect of the invention, the first data set comprising pre-acquired medical data is stored in a data storage medium in the IT-environment of the health care provider, wherein the first data set is provided as input data to the first algorithm component hosted in the IT-environment of the health care provider. Thus, respective rules and regulations of various jurisdictions for patient data protection can be observed.

According to a further aspect of the invention, the first algorithm component comprises at least an input layer and an output layer of the machine learning algorithm. In this fashion, only the IT environment of the healthcare provider processes protected patient data and outputs the determined medical decision support data.

According to a further aspect of the invention, the first algorithm component comprises at least an input layer, a subsequent layer connected to the input layer and an output layer of the machine learning algorithm. Therefore, based on the data transmitted to the second algorithm component, no conclusions can be drawn about the patient medical data at the IT service provider's site.

According to a further aspect of the invention, the first algorithm component comprises an input layer or an input layer and a subsequent layer connected to the input layer, and wherein the second algorithm component comprises all subsequent layers, in particular all subsequent hidden layers, and an output layer of the machine learning algorithm.

The IT environment of the IT service provider thus provides a main medical data processing system even though the IT service provider is not authorized to process protected patient data.

According to a further aspect of the invention, the first algorithm component transmits the outputs of its input layer or the outputs of the subsequent layer connected to its input layer to the second algorithm component via the data interface. The partitioning of the algorithm components is such that the IT service provider does not have direct access to the protected patient data.

According to a further aspect of the invention, the second algorithm component receives the outputs transmitted from the first algorithm component, wherein the second algorithm component comprises all layers of the machine learning algorithm between the input layer or the subsequent layer connected to the input layer and the output layer of the machine learning algorithm.

The division of the algorithm components is hence designed in such a way that the healthcare provider is not able to reconstruct the entire AI algorithm using reverse engineering methods or use it without authorization.

According to a further aspect of the invention, the second algorithm component transmits the outputs of its final layer to the output layer of the machine learning algorithm comprised by the first algorithm component via the data interface.

The AI algorithm is thus basically divided in such a way that at least the input and output layers of the AI algorithm are operated at the healthcare provider and are hence not accessible to the IT service provider.

According to a further aspect of the invention, the pre-acquired medical data is at least one of an ECG and/or IEGM signal curve, a respiratory signal curves, in particular a respiratory rhythm and/or a respiratory depth, a pressure waveform signal curve, a temperature waveform signal curve, a tissue and body inductance signal curve and a blood flow signal curve. The present invention is thus advantageously usable with a wide variety of data sources.

According to a further aspect of the invention, the medical decision support data is a medical parameter and/or a designation of a medical condition. The machine learning algorithm could e.g. identify patterns in ECG or other signals on the basis of which medical support data can be generated.

According to a further aspect of the invention, the machine learning algorithm is an artificial neural network, in particular a regression or classification algorithm. The machine learning algorithm can thus be applied to a broad range of problems.

According to a further aspect of the invention, training data, in particular data vectors for weights of propagation functions and threshold values of activation functions of individual neurons, for the first algorithm component and the second algorithm component is exchanged bidirectionally via the data interface. The partitioned structure of the machine learning algorithm thus enables the exchange of training data between the respective algorithm components.

The herein described features of the system for providing medical decision support are also disclosed for the computer-implemented method for providing medical decision support and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a diagram of a system for providing medical decision support according to a preferred embodiment of the invention;
- Fig. 2: shows a flowchart of a computer implemented method for providing medical decision support according to the preferred embodiment of the invention;
- Fig.3: shows a flowchart of a computer implemented method for providing medical decision support according to a further preferred embodiment of the invention; and
- Fig.4: shows a flowchart of a computer implemented method for providing a trained machine learning algorithm configured to provide medical decision support according to the preferred embodiment of the invention.

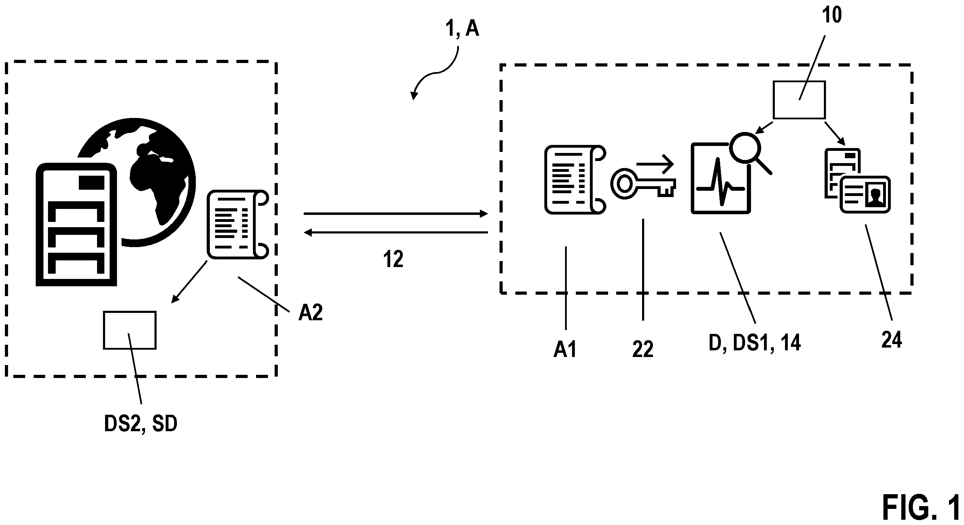

The system 1 shown in Fig. 1 comprises at least one sensor of a medical device 10 providing a first data set DS1 comprising pre-acquired medical data D.

Furthermore, the system 1 comprises means for applying a machine learning algorithm A, in particular an artificial neural network, to the pre-acquired medical data, wherein the machine learning algorithm A comprises a first algorithm component A1 hosted in an IT-environment of a health care provider and a second algorithm component A2 hosted in an IT-environment of an IT-service provider, said first algorithm component A1 and said second algorithm component A2 being linked via a data interface 12.

In addition, the system 1 comprises means for outputting a second data set DS2 representing medical decision support data SD. The machine learning algorithm A was initially developed and trained as an overall algorithm. A trained neural network is used here as an overall algorithm.

Since this algorithm needs to access protected patient data for operation, according to the invention, the algorithm is divided into two parts for operation, with the first algorithm component A1 needing access to patient data implemented in the protected IT system of the healthcare provider.

Furthermore, the first algorithm component A1 uses its input interface to access the pre-acquired medical data D and the patient medical record 24 via a secure connection 22. The results of the first algorithm component A1 are continuously made available to the second algorithm component A2 via the data interface 12.

The second algorithm component A2 is executed on the IT service provider's servers and is not directly available to the healthcare provider. With a suitable choice of splitting the neural network into two subcomponents, it is thus possible that neither the protected patient data need to be available to the AI service provider, nor can unauthorized healthcare providers use the AI algorithm without authorization from the AI service provider or spy on it by means of reverse engineering. Further training of the neural network, even during operation, also remains possible with this system division.

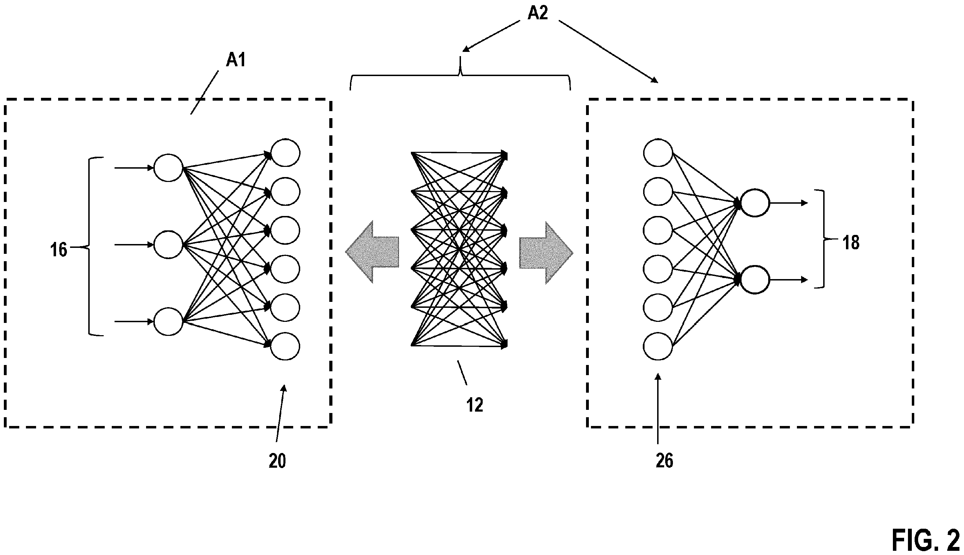

Fig. 2 shows a flowchart of a computer implemented method for providing medical decision support according to a preferred embodiment of the invention.

The machine learning algorithm A consists of a trained neural network that is given a medical input. This is then received and processed by means of an input layer 16 of suitable dimension. The weighted propagation functions pass information to a first hidden layer with appropriate activation functions and trained thresholds.

Subsequently, the information is passed to another hidden layer, again via weighted propagation functions. This further hidden layer is linked via further propagation functions to an output layer 18, which provides a classification result. The representation of the neural network is only schematic and can have many more dimensions and layers in an actual implementation.

The first data set DS1 comprising pre-acquired medical data D is stored in a data storage medium 14 in the IT-environment of the health care provider. The first data set DS1 is provided as input data to the first algorithm component A1 hosted in the IT-environment of the health care provider.

The first algorithm component A1 transmits the outputs of its input layer 16 or the outputs of the subsequent layer 20 connected to its input layer 16 to the second algorithm component A2 via the data interface 12.

The second algorithm component A2 receives the outputs transmitted from the first algorithm component A1, wherein the second algorithm component A2 comprises all layers of the machine learning algorithm A between the input layer 16 or the subsequent layer 20 connected to the input layer 16 and the output layer 18 of the machine learning algorithm A.

The pre-acquired medical data D is at least one of an ECG and/or IEGM signal curve, a respiratory signal curves, in particular a respiratory rhythm and/or a respiratory depth, a pressure waveform signal curve, a temperature waveform signal curve, a tissue and body inductance signal curve and a blood flow signal curve. The medical decision support data SD is preferably a medical parameter and/or a designation of a medical condition.

The machine learning algorithm A is an artificial neural network, in particular a regression or classification-type algorithm.

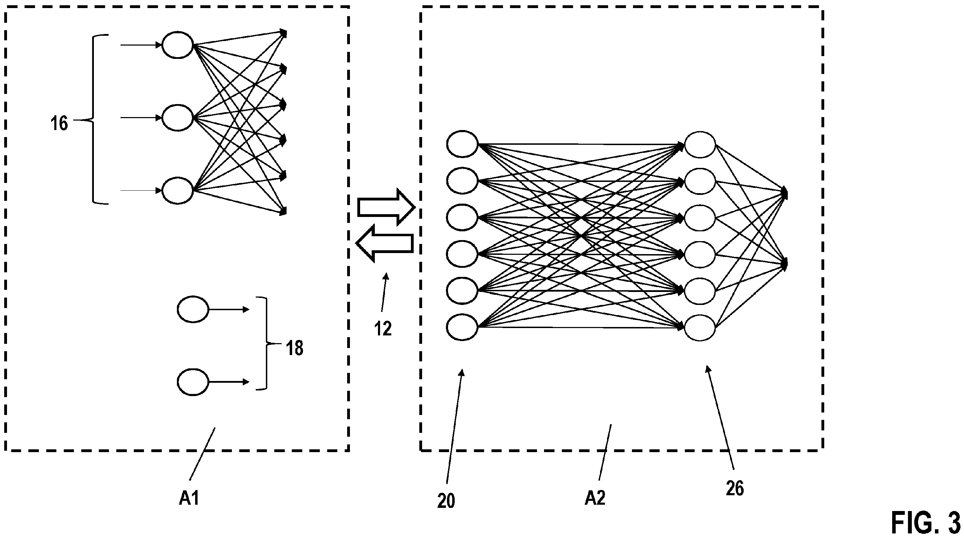

Fig. 3 shows a flowchart of a computer implemented method for providing medical decision support according to a further preferred embodiment of the invention.

The first algorithm component A1 according to this alternative embodiment comprises an input layer 16 and the output layer 18 or alternatively an input layer 16, a subsequent layer 20 connected to the input layer 16 and the output layer 18. The second algorithm component A2 comprises all subsequent and hidden layers 20, 26 of the machine learning algorithm A.

The second algorithm component A2 furthermore transmits the outputs of its final hidden layer 26 of the machine learning algorithm A to the output layer 18 of the machine learning algorithm A comprised by the first algorithm component A1 via the data interface 12.

Hence, both the input layer 16 and the output layer 18 are operated in the IT system of the healthcare provider. All other hidden layers are operated in the IT system of the IT service provider. The advantage of this division is that both access to patient data and reporting takes place exclusively in the protected IT environment of the healthcare provider.

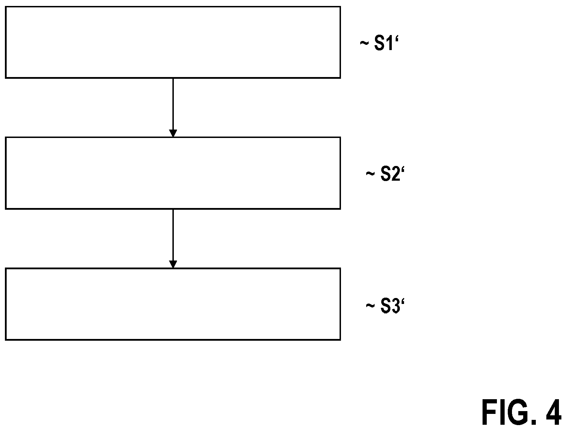

Fig. 4 shows a flowchart of a computer implemented method for providing a trained machine learning algorithm A configured to provide medical decision support according to the preferred embodiment of the invention.

The computer implemented method for providing a trained machine learning algorithm A is configured to provide medical decision support.

The method comprises receiving S1' a first training data set comprising pre-acquired medical data D captured by at least one sensor of a medical device 10.

The method further comprises receiving S2' a second training data set representing medical decision support data SD and training S3' the machine learning algorithm A by an optimization algorithm which calculates an extreme value of a loss function for determining medical decision support data SD from the pre-acquired medical data D.

Furthermore, training data, in particular data vectors for weights of propagation functions and threshold values of activation functions of individual neurons, for the first algorithm component A1 and the second algorithm component A2 is exchanged bidirectionally via the data interface 12.

### Reference Signs

- 1: system
- 10: medical device
- 12: data interface
- 14: data storage medium
- 16: input layer
- 18: output layer
- 20: subsequent layer
- 22: secure connection
- 24: patient medical record
- 26: final hidden layer
- A: machine learning algorithm
- A1: first algorithm component
- A2: second algorithm component
- D: medical data
- DS1: first data set
- DS2: second data set
- SD: medical decision support data
- S1'-S3': method steps

## Claims

1. Computer-implemented method for providing medical decision support, comprising the steps of:
providing (S1) a first data set (DS1) comprising pre-acquired medical data (D) captured by at least one sensor of a medical device (10);
applying (S2) a machine learning algorithm (A) to the pre-acquired medical data (D), wherein the machine learning algorithm (A) comprises a first algorithm component (A1) hosted in an IT-environment of a health care provider and a second algorithm component (A2) hosted in an IT-environment of an IT-service provider, said first algorithm component (A1) and said second algorithm component (A2) being linked via a data interface (12); and
outputting (S3) a second data set (DS2) representing medical decision support data (SD) by the machine learning algorithm (A).

2. Computer-implemented method of claim 1, wherein the first data set (DS1) comprising pre-acquired medical data (D) is stored in a data storage medium (14) in the IT-environment of the health care provider, wherein the first data set (DS1) is provided as input data to the first algorithm component (A1) hosted in the IT-environment of the health care provider.

3. Computer-implemented method of claim 1 or 2, wherein the first algorithm component (A1) comprises at least an input layer (16) and an output layer (18) of the machine learning algorithm (A).

4. Computer-implemented method of claim 1 or 2, wherein the first algorithm component (A1) comprises at least an input layer (16), a subsequent layer (20) connected to the input layer (16) and an output layer (18) of the machine learning algorithm (A).

5. Computer-implemented method of claim 1 or 2, wherein the first algorithm component (A1) comprises an input layer (16) or an input layer (16) and a subsequent layer (20) connected to the input layer (16), and wherein the second algorithm component (A2) comprises all subsequent layers, in particular all subsequent hidden layers, and an output layer (18) of the machine learning algorithm (A).

6. Computer-implemented method of any one of claims 3 to 5, wherein the first algorithm component (A1) transmits the outputs of its input layer (16) or the outputs of the subsequent layer (20) connected to its input layer (16) to the second algorithm component (A2) via the data interface (12).

7. Computer-implemented method of claim 3 or 4, wherein the second algorithm component (A2) receives the outputs transmitted from the first algorithm component (A1), wherein the second algorithm component (A2) comprises all layers of the machine learning algorithm (A) between the input layer (16) or the subsequent layer (20) connected to the input layer (16) and the output layer (18) of the machine learning algorithm (A).

8. Computer-implemented method of claim 7, wherein the second algorithm component (A2) transmits the outputs of its final layer to the output layer (18) of the machine learning algorithm (A) comprised by the first algorithm component (A1) via the data interface (12).

9. Computer-implemented method of any one of the preceding claims, wherein the pre-acquired medical data (D) is at least one of an ECG and/or IEGM signal curve, a respiratory signal curves, in particular a respiratory rhythm and/or a respiratory depth, a pressure waveform signal curve, a temperature waveform signal curve, a tissue and body inductance signal curve and a blood flow signal curve.

10. Computer-implemented method of any one of the preceding claims, wherein the medical decision support data (SD) is a medical parameter and/or a designation of a medical condition.

11. Computer-implemented method of any one of the preceding claims, wherein the machine learning algorithm (A) is an artificial neural network, in particular a regression or classification-type algorithm.

12. Computer implemented method for providing a trained machine learning algorithm (A) configured to provide medical decision support, comprising the steps of:
receiving (S1') a first training data set comprising pre-acquired medical data (D) captured by at least one sensor of a medical device (10);
receiving (S2') a second training data set representing medical decision support data (SD); and
training (S3') the machine learning algorithm (A) by an optimization algorithm which calculates an extreme value of a loss function for determining medical decision support data (SD) from the pre-acquired medical data (D).

13. Computer-implemented method of claim 12, wherein training data, in particular data vectors for weights of propagation functions and threshold values of activation functions of individual neurons, for the first algorithm component (A1) and the second algorithm component (A2) is exchanged bidirectionally via the data interface (12).

14. System (1) for providing medical decision support, comprising:
at least one sensor of a medical device (10) providing (S1) a first data set (DS1) comprising pre-acquired medical data (D);
means for applying (S2) a machine learning algorithm (A), in particular an artificial neural network, to the pre-acquired medical data (D), wherein the machine learning algorithm (A) comprises a first algorithm component (A1) hosted in an IT-environment of a health care provider and a second algorithm component (A2) hosted in an IT-environment of an IT-service provider, said first algorithm component (A1) and said second algorithm component (A2) being linked via a data interface (12); and
means for outputting (S3) a second data set (DS2) representing medical decision support data (SD).

15. Computer program with program code to perform the method of any one of claims 1 to 13 when the computer program is executed on a computer.
